# EUROPEAN PATENT SPECIFICATION

(11) **EP 1 651 194 B1**
(45) Date of publication and mention of the grant of the patent: **31.03.2010**
(21) Application number: 03817894.3
(22) Date of filing: 06.08.2003
(51) Int. Cl.: A61K 9/36, A61K 9/52

(54) **STABLE CONTROLLED RELEASE PHARMACEUTICAL COMPOSITIONS CONTAINING FENOFIBRATE AND PRAVASTATIN**
STABILE PHARMAZEUTISCHE ZUBEREITUNGEN ZUR KONTROLLIERTEN FREISETZUNG VON FENOFIBRAT AND PRAVASTATIN
COMPOSITIONS PHARMACEUTIQUES STABLES A LIBERATION CONTROLEE CONTENANT DU FENOFIBRATE ET DE LA PRAVASTATINE

(43) Date of publication of application: 03.05.2006
(73) Proprietor: Galephar M/F, 6900 Marche-en-Famenne (BE)
(72) Inventor: VANDERBIST, Francis, B-1650 Beersel (BE); BAUDIER, Philippe, B-1180 Uccle (BE); DEBOECK, Arthur, Gurabo, Puerto Rico 00778 (US); SERENO, Antonio, B-1820 Melsbroek (BE)
(74) Representative: Vandeberg, Marie-Paule L.G.
(86) International application number: PCT/BE2003/000133
(87) International publication number: WO 2005/013940

(56) References cited:
- WO-A-02/076376
- WO-A-03/013501
- WO-A-03/013608
- US-A1- 2002 161 032
- US-A1- 2003 049 314
- US-B2- 6 368 620

## Description

The present invention is aimed at a novel pharmaceutical preparation useful for the treatment of hypercholesterolemia and/or hyperlipidemia. Disclosed are pharmaceutical dosage forms containing a statin and a fibrate. In particular, the invention relates to a single unit dose formulation, containing a mixture of Pravastatin and Fenofibrate.

The invention relates to a stabilised formulation containing Pravastatin. In particular the Pravastatin is stabilised by blending and/or granulating the Pravastatin with an alkaline substance which imparts a pH of less than 9 to an aqueous dispersion and/or solution of said composition.

The alkaline substance is sodium bicarbonate.

The present invention also discloses a single unit dose formulation which releases the Pravastatin or its acceptable pharmaceutical salts and the Fenofibrate at a predefined controlled manner. The formulation is characterised in vivo in such a manner that the absolute value of the differences between the Fenofibric acid (the active metabolite of fenofibrate) and Pravastatin time to maximum concentration (Tₘₐₓ) after single dose administration is at least 1.5 hour, advantageously at least 2 hours.

### BACKGROUND OF THE INVENTION

Hypercholesterolemia is a main player in the development of atherosclerosis diseases in general and coronary heart diseases in particular. The risk of progression of the atherosclerosis process to coronary heart diseases increases progressively with increasing levels of total serum cholesterol, low density lipoproteins (LDL) cholesterol and triglycerides at both the individual and the population level. Drugs used to treat hypercholesterolemia pertain mainly to the family of statins and fibrates.

The statin family of drugs also called 3-Hydroxy-3Methylglutaryl-Coenzyme A (HMG-CoA) reductase inhibitors are reversible inhibitors of the microsomal enzyme HMG-CoA reductase, which converts HMG-CoA to mevalonate which is a precursor in the cholesterol biosynthesis. Inhibition of HMG-CoA reductase by statins decreases intracellular cholesterol biosynthesis, which then leads to transcriptionnally upregulated production of microsomal HMG-CoA reductase at cell surface LDL receptors.

Subsequently, additional cholesterol is provided to the cell by de novo synthesis and by receptor-mediated uptake of LDL-cholesterol from the blood. This resets intracellular cholesterol homeostasis in extrahepatic tissues, but has little effect on the overall cholesterol balance (Clin. Pharmacokinet. 1997, May, 32(5), 403-425).

The most important molecules belonging to the statins family are: pravastatin, simvastatin, atorvastatin, fluvastatin and lovastatin. They differ by their physico-chemical properties and by their pharmacokinetic properties.

Pravastatin sodium produces its lipid-lowering effect in two ways. First, as a consequence of its reversible inhibition of HMG-Co4 reductase activity, it effects modest reductions in intracellular pools of cholesterol. This results in an increase in the number of LDL-receptors on cell surfaces and enhanced receptor-mediated catabolism and clearance of circulating LDL. Second, pravastatin inhibits LDL production by inhibiting hepatic synthesis of very low-density lipoproteins (VLDL), the LDL precursors.

Pravastatin slows the progression of atherosclerosis in humans and may have beneficial effects in stabilizing plaques, improving endothelial dysfunction, decreasing platelet thrombus formation, improving fibrinolytic activity and reducing incidence of transient myocardial ischaemia.

Fenofibrate belongs to another class of hypocholesterolemic agents: the fibrates. The lipid-modifying effects of fenofibrate are mediated via the activation of the peroxisome proliferator-activated receptors (PPARs).

Fenofibrate or p-(4-chlorobenzoyl)-phenoxy isobutyrate isopropyl ester is useful for the treatment of adult patients with very high elevations of serum triglyceride levels and/or cholesterol levels. The usual daily dosage is 100 to 300mg which is administered in one or two doses. Fenofibrate absorbed as fenofibric acid, resulting from the hydrolysis of fenofibrate, is extensively bound to plasma albumin. The plasma half-life is about 20 hours. Fenofibric acid is excreted predominantly in the urine, mainly as the glucuronide conjugate, but also as a reduced form of fenofibric acid and its glucuronides.

Fenofibrate reduces plasma total cholesterol (TC), low-density lipoprotein cholesterol (LDL-C), triglyceride (TG) and very-low-density lipoprotein (VLDL) cholesterol levels, and increases high-density lipoprotein cholesterol (HDL-C) and apolipoprotein (Apo) Al and Apo All levels in patients with dyslipidaemia.

Fenofibrate also reduces plasma fibrinogen levels in both normolipidemic individuals and those with dyslipidemia, and is significantly more effective in that reduction than simvastatin, atorvastatin or pravastatin. This is of significance since increased levels of fibrinogen or plasminogen activator inhibitor (PAl-1) are associated with an increased risk of atherosclerosis and coronary heart disease (CHD).

Fenofibrate has also demonstrated a very important activity in reducing the levels of the inflammatory marker C reactive protein (CRP). The C-reactive protein has been recognized to have a negative effect on the evolution of the pathogenesis of artherosclerosis and coronary heart diseases.

Although each of Pravastatin and Fenofibrate alone have demonstrated their ability to reduce levels of cholesterol in large amount of patients with hypercholesterolemia. There still remains an important number of patients that fail to reach the desired cholesterol levels Moreover the number of patients failing to reach the desired cholesterol levels dramatically increased when the cholesterol upper limit levels guidance were reduced from 250mg/dL to 200mg/dl. This situation will further worsen since the actual trend is to lower these limits even more to less than 150mg/dl. Patients with increased difficulty to lower their high cholesterol levels include but are not limited to diabetic patients, patients having the metabolic syndrome and patients with mixed severe hyperlipidemia. For all those patients, a combination of a statin with a fibrate would definitely be beneficial in order to normalise their cholesterol levels.

While such combination would be very effective in reducing the cholesterol levels, it shall be remembered that the co-administration of a statin and a fibrate is not without potential serious side effects that may lead to patient's death. Therefore it is remembered that the co-administration of the statin cerivastatin (Baycol®) with the fibrate gemfibrozil was responsible for the death of tenth of patients. This situation obliged the USA Food and Drug Administration to interdict the use of such combination. The inventors of the present invention have overcome this problem by controlling the release of the statin (Pravastatin) and the fibrate (Fenofibrate) in such a manner that the maximum blood concentrations of each of the drug does not appear at about the same time after the administration. In particular the absolute value of the differences between the Fenofibric acid and the Pravastatin plasma levels time to maximum concentration (Tₘₐₓ) after single does administration is at least 2 hours.

To increase the safety of the combination product it is essential that the two components (Fibrate and Statin) are taken at very precise predefined times. The invention of the present invention have overcome this problem by combining the two drugs into a single unit dose that contains both drugs with controlled release profile and to be taken once a day.

The therapeutic effect of concomitant administration of fibrates and statins has been evaluated in several clinical studies. For instance, Ellen and al (1998) demonstrated that the combination of fenofibrate combined to either pravastatin 20 mg or simvastatin 20 mg was an effective and safe treatment of combined hyperlipidemia. Feber and al (1995) have assessed the long-term safety of combinations of pravastatin and simvastatin with either bezafibrate or fenofibrate for up to three years and have concluded that the combinations of drugs assessed were not associated with serious disturbances in biochemical markers of liver or muscle function. Shek and Ferill (2001) have reviewed the literature concerning clinical trials performed with fibrate-statins combinations and concluded that this kind of combination therapy offers significant advantages for the treatment of severe or refractory mixed hyperlipidemia. They observed that the risk of myopathy is lower with this kind of combination than with higher doses of statins alone. Farnier and al (2000) have compared the administration of fluvastatin + fenofibrate with fenofibrate) in patients with severe primary hypercholesterolemia and conclude that the combination treatment resulted in substantial improvement in atherogenic plasma lipids and is well tolerated. Finally, Athyros and al have assessed the efficacy and safety of a combination of atorvastatin + fenofibrate versus each monotherapy in patients with type 2 diabetes combined with hyperlipidemia. They concluded that the combination was highly beneficial on all lipid parameters and improves patient's coronary artery disease risk status significantly more than each drug alone. So it clearly appears that the combination treatment seems interesting to treat some categories of patients with hyperlipidemia. Nevertheless, it should be noted that all studies mentioned hereinabove were performed by administrating separately the fibrate compound and the statin compounds and therefore failed to provide for a precise control of the pharmacokinetic profile of both drugs and mainly failed to provide for an at least 2 hours differences in the time to maximum (Tₘₐₓ) of each of the statin and the fenofibric acid after single oral dose.

European Patent Application 455,042 describes a method for treating dyslipidemia by administering a combination of Pravastatin and a fibric acid derivative and European Patent Application 475,148 describes the use of Pravastatin alone or in combination with a fibric acid derivative for the preparation of a pharmaceutical preparation useful in preventing or treating Type III hyperlipoproteinemia. Both patents while disclosing the use of Pravastatin and a fibric acid derivatives in combination fail to disclose the need for the difference of time of maximum blood concentration between the 2 active components and also never described is a composition able to deal with the multiple problems linked to fibrate / statin combination.

A pharmaceutical preparation, to be useful must show acceptable stability properties. This is, to be practical show stability for at lest 2 years under the standard ICH regulations. Furthermore, Pravastatin, a 4-hydroxyalkylacid, forms easily upon storage a lactone by losing a molecule of water. The levels of lactone must be kept very low.

US patent application (20020161032) claims a composition containing fenofibrate and a statin wherein the fed-fasted effects were reduced thanks to the use of a phospholipid as a surface active substance. US patent 6,180,660 describes a method for preventing cardio-vascular diseases using a HMG-CoA reductase inhibitor alone or in combination with another lipid altering agents in subjects with specific cholesterol levels.

Nevertheless, these 2 patents fail to disclose how to stabilize pravastatin and to avoid side effects linked to simultaneous high blood levels of both active ingredients.

The WO 01/37831 describes a pharmaceutical combination comprising separate dosage form in a common blister card of an inhibitor of the HMG-CoA reductase and a fibric acid derivative useful in the treatment of dyslipidemia of diabetics and non-diabetics. Since this application does not describe a single unit dose, it cannot control the relative apparition of the times of maximum concentration of both drugs.

The stability of statins from the family of the 4 hydroxy alkylacid, such as Pravastatin, are a known challenge for the pharmaceutical formulator. Indeed, various environmental factors such as temperature, moisture, low pH, carbon dioxide may cause significant degradation of these compounds.

US Patents 5,030,447 and 5,180,589 describe pharmaceutical compositions in the form of a tablet with enhanced stability comprising Pravastatin characterised in that it contains one or more basifiying agent to impart a pH of at least 9 to an aqueous solution and/or dispersion of said composition. The use of alkalinising substances who give a pH above 9 is certainly not recommended for the patient stomach and lower pH values, less than 9, which are more physiological are certainly preferred.

US patent 6,531,507 describes a composition containing a statin compound obtained by a process of co-crystallisation and/or co precipitation of the statin compound with a buffering or a basifying substance. This patent describes statin, pure active drug, stabilisation by a process of co-crystallisation and/or co-precipitation that involves solubilising the statin into organic solvent. This process, while very expensive, addresses the stability of the statins presented under the form of pure chemical substances.

WO 03/013608 A describes the combination of a semi-solid formulation of fenofibrate with a pravastatin tablet coated with a water-soluble material.

Nevertheless, it fails to disclose the stabilisation of a pharmaceutical formulation containing Pravastatin by a process which is cost effective.

### Field of the invention

The present invention is aimed at a novel pharmaceutical preparation according to claim 1 useful for the treatment of hypercholesterolemia and/or hyperlipidemia.

The combination of a HMG-CoA reductase inhibitor and fenofibrate (administered in two separate dosage forms) is known to be better tolerated and equally if not more efficient as a higher dose of the HMG-CoA reductase inhibitor derivatives in the treatment of hypercholesterolemia because both drugs have complementary and even synergistic mechanism of action as shown in the table hereinbelow

**Table : effect of fibrates and statins on the different lipid parameters**

| **Effect** | **Statin** | **Fibrate** |
|---|---|---|
| LDL decrease | +++ | + |
| TG-rich lipoprotein decrease | + | +++ |
| HDL increase | + | ++ |
| Postprandial lipemia decrease | ± | ++ |
| Improvement in LDL size profile | ± | ++ |
| Prevention of lipoprotein oxidation | ++ | ± |
| Pleiotropic effects | +++ | + |
| HDL - high density lipoprotein; LDL - low density lipoprotein; | | |
| TG - triglyceride. | | |

Some clinicians already prescribe to the same patients separate forms of a statin and of a fibrate. This separate administration has the disadvantage to complicate the posology for the patients, to increase the risk of mistakes or omissions of the drugs intake and does not provide for a precise control of the pharmacokinetics of the active substances which leads to the reduction of the risk of muscular side effects.

Useful formulation should be easy to take, efficacious, reduced side effects and stable. Disclosed are once a day single unit dose formulation containing both Pravastatin and Fenofibrate. The complementary and/or synergistic mechanism of action of Pravastatin and Fenofibrate are known and will render the formulation very attractive to reduce the cholesterol levels in patients in need. The controlled release characteristic of the formulation resulting in the separation in time of the maximum plasma concentration of both drugs will maintain the risk of side-effect of said preparation very low.

Also described is a pharmaceutical composition containing pravastatin and fenofibrate suitable for once daily administration that can be taken in the morning or in the evening.

Also described is a stabilised pharmaceutical formulation.

It is an object of the present invention to combine in a single dose form a statin, which is pravastatin, and a fibrate, which is fenofibrate. These drugs have the important advantage to possess complementary mechanisms of action. The pharmacological effects of this combination is to act efficiently on the usual lipid triad : LDL, HDL, TG and inflammatory markers like CRP (c-reactive protein).

Also described is a pharmaceutical composition comprising a combination of fibrates and statins which is safe. A particular safety concern is the potential for the occurrence of myopathy. Indeed, both kinds of drugs can provoke muscular side effects, leading in the worst case to fatal rhabdomyolysis. Rhabdomyolysis has for instance been observed when cerivastatin was combined to gemfibrozil and resulted in the removal from the market place of Cerivastatin containing drug product.

Also described is a very efficient hypocholesterolemic treatment to patients in such a need while reducing the risk of side-effects.

It is also described to combine into the same pharmaceutical dosage form a statin and a fibrate which are eliminated by different routes of metabolisation. Avoiding the risk of any direct metabolization interactions between the two drugs of the combination. Indeed, pravastatin is considered as a particularly safe statin since this molecule is not metabolised through the cytochrome P450 system, what minimizes the risk of interactions with other drugs or with food. On the other hand, fenofibrate is not metabolised by cytochrome P450 3A4, the isoenzyme of the cytochrome P450 system involved in the metabolization of the majority of drugs and is only weakly metabolised by cytochrome P450 2C9 isoenzyme (Martindale 2000).

Also described is the use of pravastatin, a statin which takes its main activity from the parent drug and not from active metabolites. Also, the only active metabolite has pharmacokinetic properties very close to those of the parent drug.

It is also described to provide a pharmaceutical composition useful for the treatment of hyperlipidemia comprising Pravastatin or one of its pharmaceutical acceptable salts and Fenofibrate characterised that the absolute difference in time to maximum concentration (Tₘₐₓ)of Fenofibric acid and Pravastatin after single dose administration with food is at least 2 hours.

It is an object of the present invention to provide for a pharmaceutical composition consisting in a capsule containing the Fenofibrate and the Pravastatin. The capsule can be any type hard or soft gelatin, and/or Hydroxypropyl Methhylcellulose capsules.

Another object of the present invention is that the Fenofibrate may be present in the pharmaceutical dosage form under the form of micronized powder, micronized Fenofibrate coated onto a carrier and / or beads, co-micronized Fenofibrate with a surfactant such as sodium lauryl sulfate, dispersed and/or dissolved Fenofibrate in polyglycerides, oils, surfactant and mixtures thereof.

The fenofibrate can be formulated as powder to be filled into capsules for instance by micronized active ingredient or co-micronized active ingredient with a surfactant mixed, for instance, with some of the following excipients : pregelatinized starch, lactose, mannitol, magnesium stearate, sacchararose, magnesium stearyl fummarate, silice colloidal or talc.

Fenofibrate can also be dispersed or dissolved in fatty excipients like , but not restricted to polyglycolized glycerides, polyethylene glycols, vegetal or mineral oils, derivatives of fatty acids, medium chain triglycerides. Other non-fatty excipients can be used to improve those formulations like hydroxypropylcellulose, ascorbyl palmitate, antioxydants.

Those suspensions or solutions of fenofibrate in excipients can be filled into hard gelatin or HPMC capsules or used to coat inert beads (for instance sugar or starch) with the said solution or suspension. Fenofibrate can also be granulated with the fatty granules mentioned hereinabove, to obtain either beads or fatty granules of fenofibrate, which can then be compressed or filled into tablets. Fenofibrate beads require the presence in the composition of other excipients allowing the spheronization such as for instance microcrystalline celullose, lactose, other cellulose derivative, polyvinylpyrrolidone, sucrose stearate.

It is also also described to provide Fenofibrate pharmaceutical composition presenting modified release profiles such as: immediate release, delayed release, extended release and any combination thereof.

It is also described to provide a stabilised Pravastatin, pharmaceutical composition under the form of Pravastatin sodium salt.

It is also described to provide the Pravastatin sodium salt under the form of powder, tablet, beads obtained by extrusion spheronization, beads obtained by coating inert or active support.

Powder of pravastatin can be obtained by direct mixing of the active ingredient with for example lactose monohydrate, mannitol, sodium bicarbonate, calcium hydrogenophosphate, silice colloidal, magnesium stearate or magnesium stearyl fumarate. Powder of pravastatin can also be obtained by granulation of the active ingredient with lactose, sodium bicarbonate, calcium hydrogenophosphate, mannitol, microcrystalline celullose, polyvinylpyrollidone, sodium croscarmellose, polyplasdone, sodium starch glycolate, magnesium stearate or magnesium stearyl fumarate. The powder so obtained may be filled into hard gelatin or HPMC capsules.

Tablets of pravastatin can be obtained by direct compression or wet granulation. Excipients which can be used but not restricted to for direct compression : lactose, sodium bicarbonate, calcium hydrogenophosphate, mannitol, polyvinylpyrollidone, sodium croscarmellose, polyplasdone, sodium starch glycolate, magnesium stearate or magnesium stearyl fumarate. Excipients which can be used but not restricted to for wet granulation are : lactose, sodium bicarbonate, calcium hydrogenophosphate, mannitol, microcrystalline celullose, ascorbyl palmitate polyvinylpyrollidone, sodium croscarmellose, polyplasdone, sodium starch glycolate, magnesium stearate or magnesium stearyl fumarate.

Beads of pravastatine obtained by extrusion-spheronisation may content (but are not restricted to) one or more of the following excipients : microcrystalline celullose, lactose, sodium carboxymethylcellulose, sucrose stearate, polyethyleneglycol, ascorbyl palmitate, polyvinylpyrollidone, hydroxypropylmethyl cellulose or another cellulose derivative.

Inert beads may be beads of sugar or starch or other any other pharmaceutically acceptable excipients, those beads -can for example be coated with an aqueous solution of pravastatin together with, for example, polyvinylpyrollidone, hydroxypropylmethylcellulose, polyethyleneglycol, titane oxide, talc, polysorbate (Tween 80).

Another object of the present invention is to provide Pravastatin formulation stabilised by mixing, blending and/or granulating the Pravastatin with alkaline bicarbonate with or without other pharmaceutical excipients the resulting formulation when dissolved and/or dispersed in water having a pH equal or lower than 9, the bicarbonate being sodium bicarbonate.

In another aspect the pravastatin formulation contains an antioxidant for improved stability.

The composition of the present invention can advantageously contain, in addition to pravastatin and fenofibrate and suitable excipients, ingredients allowing to control the homocystein levels of patients such as vitamins amoung the group of vitamins B, minerals or other nutrients. Folic acid, vitamin B6, vitamin B12 and mixtures thereof (especially folic acid and mixture containing folic acid) seem to be particularly suitable to control the homocystein plasma levels of patients. The composition of the present invention can also advantageously contain, in addition to pravastatin and fenofibrate and suitable excipients two or more of the said vitamins or minerals nutrients. For instance a combination of folic acid, vitamin B6 and/or Vitamin B12, can be added to the composition containing fenofibrate and pravastatin.

It is also described to provide an easy, cost effective and reliable process for manufacturing the fenofibrate-pravastatin stabilized composition: characterised in that to the capsule containing the Fenofibrate is added a tablet (coated or not) or beads (coated or not) of Pravastatin.

### DETAILED DESCRIPTION

The definition of the main pharmacokinetic parameters used in the present invention is given hereinbelow. Those definitions are taken from the "Note for guidance on the investigation of bioavailability and bioequivalence" published by the European Agency for the Evaluation of Medicinal Products (EMEA).

| | |
|---|---|
| Cₘₐₓ | Maximal plasma concentration |
| Cₘᵢₙ | Minimal plasma concentration |
| tₘₐₓ | Time passed since administration at which the plasma concentration maximum occurs |
| AUCₜ | Area under the plasma concentration curve from administration to last observed concentrations at time t. |
| t_{½} | Plasma concentration half-life. |

Fenofibrate is rapidly absorbed following oral administration. The extent of absorption varies from 30-50% when taken while fasting, but increases to 60-90% when taken after a meal. There is considerable inter-individual variation. Peak plasma levels of the principal active metabolite, fenofibric acid, following a single oral dose of 300 mg fenofibrate are in the range of 6-9.5 mg/L.

Following absorption, presystemic metabolism is virtually complete with rapid conversion of the drug to fenofibric acid (which is the active metabolite of fenofibrate) and others metabolites by intestinal and plasma esterases.

There is some evidence of a correlation between the plasma concentration of fenofibric acid and its lipid lowering effects, although the extent to which this is seen may depend on the type of hyperlipidaemia (Raslova et al, 1997).

The mean plasma half-life of fenofibric acid is 19.6 - 26.6 hours in healthy, fasting young adults; this is little altered by diet or by repeat administration. Fenofibric acid is eliminated mainly in the urine, as a conjugated derivative (fenofibric acid ester glucuronide, 60-88%). Lesser amounts of fenofibric acid and the benzhydrol and its glucuronide are also found in the urine.

There is no evidence of fenofibric acid accumulation following repeated fenofibrate administrations.

Pravastatin sodium is administered orally in the active form. In clinical pharmacology studies in man, pravastatin is rapidly absorbed, with peak plasma levels of parent compound attained 1 to 1.5 hours following ingestion. Based on urinary recovery of radiolabeled drug, the average oral absorption of pravastatin is 34% and absolute bioavailability is 17%. (Singhvi et al, 1990; Sasahara et al, 1988)

While the presence of food in the gastrointestinal tract reduces systemic bioavailability, the lipid-lowering effects of the pravastatin are similar whether taken with, or 1 hour prior, to meals. (Pan et al, 1993)

Systemic bioavailability of pravastatin administered following a bedtime dose was decreased by 60% compared to that following morning administration. Despite this decrease in systemic bioavailability, the efficacy of pravastatin administered once daily in the evening, although not statistically significant, was marginally more effective than that after a morning dose. This finding of lower systemic bioavailability suggests greater hepatic extraction of the drug following the evening dose. (Triscari et al, 1991)

Biotransformation of pravastatin, contrary to other drugs of the same class (simvastatin, atorvastatin) does not involve the cytochrome P450 system.

In contrast to other HMG-CoA reductase inhibitors, which depend on their metabolites for their pharmacological activity, 75% of the inhibitory activity of pravastatin is attributable to the parent drug.

Steady-state AUCs, Cₘₐₓ and Cₘᵢₙ plasma concentrations do not show evidence of pravastatin accumulation following once or twice daily administration of pravastatin sodium tablets.

The terminal plasma elimination half-life (t_{1/2β}) of pravastatin ranges from 1.3 to 2.6 hours in both healthy volunteers and patients with hypercholesterolemia. (Pan et al, 1987; Pan et al, 1990; Sasahara et al, 1988)

Corresponding values for its major metabolite are 0.8 to 1.3 hours. (Pan et al, 1990)

The use of Pravastatin has been associated with severe myopathy, including rhabdomyolysis side effects which are directly proportional to the dose administered.

The importance of statin side effects became extremely visible during the year 2001 when in August, Baycol^{®} (Cerivastatin) had to be removed from the market place following reports of rhabdomyolysis, including related deaths. Cerivastatin revealed an increasing reporting rate of rhabdomyolysis especially when co-administrated with gemfibrozil (a fibrate)

Nevertheless, to reduce to the maximum the potential risk of side-effects, it would be highly desirable to dispose of a combination product with adequate Controlled Release pharmacokinetic properties i.e. a drug product which present the property of giving, after oral administration to humans, mean respective time to plasma peaks (Tₘₐₓ) of each drug sufficiently separated, in order that the plasma level of the first drug released is already significantly decreased when the mean Tₘₐₓ of the second drug occurs. The present invention discloses a composition combining pravastatin and fenofibrate in a single dosage unit, which upon intake provides different absorption characteristic for each of the drug.

In a preferred embodiment, the present invention discloses a composition containing Pravastatin and Fenofibrate in a single dosage unit which upon intake provides a rapid Tmax of pravastatin and a longer Tₘₐₓ of fenofibrate in such a way that the mean plasma concentration of pravastatin is significantly decreased when mean Tₘₐₓ of fenofibric acid occurs. In this manner, high plasma concentrations of both drugs are never reached simultaneously and the risk of side effects, which is proportional to plasmatic concentration of each drug, is minimised.

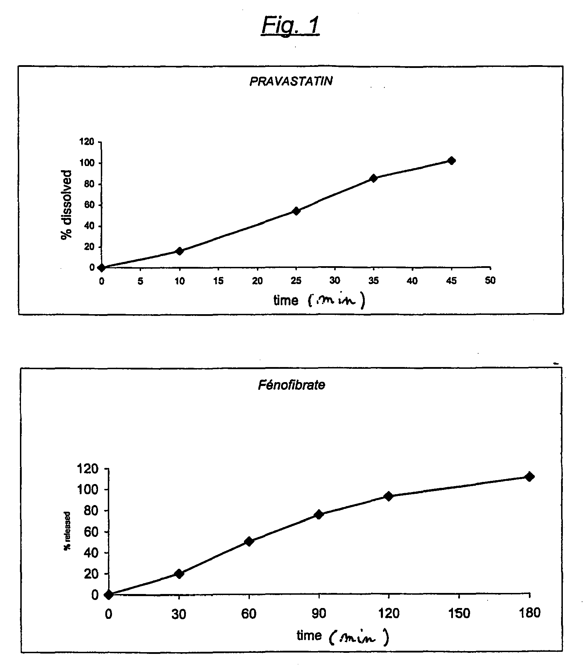

On the other hand the Tₘₐₓ value for each of the drugs should be as separated as possible and ideally the absolute difference between the Tₘₐₓ of fenofibric acid and the Tmax of pravastatin shall not be less than 1.5 hours, preferably not less than 2.0 hours. An example of a desired pharmacokinetic profile is described in figure 1 corresponding to plasma profile of both drugs after oral administration of the combination fenofibrate 160 mg + pravastatin 20 mg.

The obtention of such pharmacokinetic profiles is very challenging since the bioavailability of fenofibrate (and hence fenofibric acid) is significantly affected by the intake of food while the bioavailability of pravastatin is almost unaffected. Consequently, to present an adequate pharmacokinetic profile, the combination drug should be taken preferably with food. Unfortunately, the usual effect of food intake on the pharmacokinetic profile of drugs is to delay the Tₘₐₓ and also to delay the Tₘₐₓ of pravastatin, making the reaching of the goals still more difficult. Surprisingly enough, the composition of the present invention allows to obtain a short Tₘₐₓ of pravastatin, after administration of a fenofibrate-pravastatin combination with a standard meal (continental breakfast), see figure 1.

Different pharmaceutical formulations may be used to obtain this pharmacokinetic profile as the release of pravastatin is as rapid as possible and the release of fenofibrate occurs later. For instance, a capsule containing a coated or uncoated tablet of pravastatin with a semi-solid composition of fenofibrate is suitable.

Other alternatives are capsules containing pravastatin under the form of powder, pellets or tablets and fenofibrate formulated as paste, semi-solid tablet, granulated powder or pellets, coated or uncoated tablets.

To avoid simultaneous high plasma concentrations of both drugs, the combination may release pravastatin before and/or after fenofibrate. Also a slow pravastatin release during a period of time is acceptable. To release the pravastatin before the fenofibrate immediate release formulation shall be selected. The release of pravastatin after the release of fenofibrate can be achieved with delayed release formulation while the gradual release over a period of time can be achieved with a controlled or delayed and controlled release formulation.

Details and characteristics of composition of the invention are given in the attached claims. Advantageously the statin preferably the pravastatin) is in a first solid or semi solid form which is free or substantially free of fibrate (especially fenofibrate), while the fibrate (preferably fenofibrate) is in a second solid or semi solid form which is free or substantially free of statin (especially pravastatin). Advantageously at least a coating layer forms a barrier between the first form and the second form.

Further details will also appear from the following description of examples, in which reference is made to the attached drawings.

### Brief description of the drawings

Figure 1 represents the mean dissolution values (% of compound dissolved in function of the time expressed in minute) of six vessels for the pravastatin and the fenofibrate, while
Figure 2 gives the mean plasma concentration curves versus time for fenofibric acid and pravastatin obtained after administration of the combination fenofibrate-pravastatin of example 4.

### Examples

The invention is additionally illustrated in connection with the following examples, which are considered to be illustrative of the present invention.

### Example 1

| **Ingredient Name** | **Amount [g]** |
|---|---|
| Pravastatin Sodium Salt | 20 |
| Sodium bicarbonate | 110 |
| Ascorbyl palmitate | 2 |
| Lactose monohydrate | 19 |
| Cellulose microcrystalline | 12 |
| Povidone K30 | 2 |
| Water for granulation | 25 |
| Magnesium stearate | 2 |
| Sodium starch glycolate | 13 |

Lactose, sodium bicarbonate, ascorbyl palmitate, lactose monohydrate, cellulose microcrystalline and povidone K30 were blended in a planetary mixer for about 5 to 10 minutes until an homogeneous blend was obtained. While under agitation, a solution containing the pravastatin sodium salt into the water for granulation was added to granulate the powders. The granules obtained were dried at about 40°C for about 5 hours. Thereafter the dried granules were screened through a 1.0mm sieve, and further blended into a planetary mixer for about 2 minutes after the addition of the magnesium stearate and sodium starch glycolate.
The final mix was compressed into tablets using a rotary compressing machine equipped with punches of the deep cup type with a diameter of 6.5mm. The mean weight of the tablets was 180 mg. Corresponding to tablets containing 20 mg of pravastatin sodium salt. The tablet hardness was comprised between 4 and 6 kilopascals (Kp).
When the core tablet was placed into 100ml of demineralized water a pH of about 8.2 was measured.

### Example 2

| **Ingredient Name** | **Amount [g]** |
|---|---|
| Povidone K30 | 35 |
| Talc | 35 |
| Triacetin | 5 |
| Absolute Alcohol | 300 |

This coating solution was applied to the tablets from Example 1 using a pan coater. The amount of coating applied was about 14.4mg of dry coating (weight gain) per tablet.

### Example 3

| **Ingredient Name** | **Amount [g]** |
|---|---|
| Fenofibrate powder | 160 |
| Lauroyl macrogolglyceride | 240 |
| (gelucire 44/14) | |
| Polyethylene glycol 20,000 | 48 |
| Hydroxypropylcellulose | 95.0 |
| Sodium starch glycolate | 20.0 |
| Ascorbyl palmitate | 1.0 |

Gelucire 44/14 and polyethylene glycol 20,000 were added to a mixer equipped with a double wall bowl. The mixer was started and the bowl was warmed at about 75°C. When the gelucire and the polyethylene glycols were molten, the other ingredients (Fenofibrate, hydroxypropyl cellulose, sodium starch glycolate and ascorbyl palmitate) were added while maintaining the temperature at about 70 - 75°C.

### Example 4

The combination product was obtained by filling, into size 0 elongated hard gelatin capsules, one tablet of Example 2 and 564mg of the hot blend of Example 3. After filling, the capsules were cooled by placing them on trays between 4 and 8°C.
The capsules obtained contained 20mg of pravastatin and 160mg of fenofibrate.
The capsules of Example 4 were submitted to a dissolution test using USP apparatus #2 at a speed of 100 rpm and 900 ml of an aqueous solution adjusted at pH5.5 with sodium hydroxide and containing 1.24g of potassium dihydrogeno phosphate, 27g of polysorbate 80 and 2.88g of as dissolution medium. The pravastatin amounts dissolved after 10, 25 , 35 and 45 minutes were measured by high performance liquid chromatography.
The fenofibrate amounts dissolved after 30, 60 90, 120 and 180 minutes were measured by UV spectrocopy at a wavelength of about 281nm. Figure 1 represent the mean dissolution values of six vessels for the pravastatin and the fenofibrate.
This test shows very different in vitro releases profiles for pravastatin and fenofibrate. The pravastatin is almost completely dissolve after 45 minutes while it takes about 180 minutes for the fenofibrate to be completely dissolved.

To illustrate the present invention, a pharmacokinetic study has been performed in human healthy volunteers. The combination formulation of Example 4 consisting of a capsule containing 20 mg of pravastatin in a coated tablet and 160mg of fenofibrate in a semi solid paste was administered to the six healthy volunteers following a standardized breakfast consisting of: Cold cuts, cheese, salad, bread, butter and jam or lasagna and 240ml of water.

The healthy volunteers were caucasians of both sexes aged from 18 to 50 years.

Blood samples were taken regularly up to 12 hours after administration. The drugs analysed were fenofibric acid and pravastatin. The concentrations obtained at each sampling point are shown in Table 2.

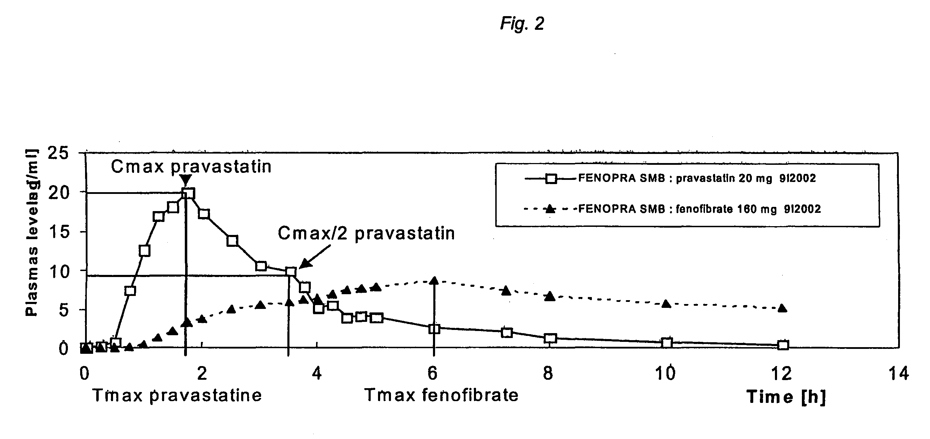

The mean plasma concentration curves versus time for fenofibric acid and pravastatin obtained after administration of the combination of example 4 are shown in Figure 2.

**Table 2: Individual plasma levels of pravastatin [ng/ml] and fenofibric acid [µg/ml] after administration of a capsule containing 160 mg of fenofibrate and 20 mg of pravastatin in 6 human volunteers. (Product of Example 4).**

| **Pravastatin Plasma Levels [ng/mL]** | | | | | | | | | | | | | | | | | | | | | | | | | | |
|---|---|---|---|---|---|---|---|---|---|---|---|---|---|---|---|---|---|---|---|---|---|---|---|---|---|---|
| samples | T0 | T1 | T2 | T3 | T4 | T5 | T6 | T7 | T8 | T9 | T10 | T11 | T12 | T13 | T14 | T15 | T16 | T17 | T18 | T19 | T20 | T21 | T22 | | | |
| Hours | 0,00 | 0,25 | 0.50 | 0,75 | 1,00 | 1,25 | 1,50 | 1,75 | 2,00 | 2,50 | 3,00 | 3,50 | 3,75 | 4,00 | 4,25 | 4,50 | 4,75 | 5,00 | 6,00 | 7,25 | 8,00 | 10,00 | 12,00 | AUCt | Cmax | Tmax |
| sub 1 | 0,0 | 0,1 | 0,1 | 0,3 | 0,6 | 3,3 | 8,3 | 8,9 | 6,8 | 7,3 | 6,8 | 3.7 | 2,6 | 2,8 | 3,1 | 2,9 | 2,6 | 3,0 | 2,0 | 1,2 | 0,6 | 0,3 | 0,5 | 27,1 | 8,9 | 1,8 |
| sub 2 | 0,0 | 0,0 | 0,0 | 0,0 | 0,1 | 0,0 | 0,1 | 0,4 | 1,4 | 4,7 | 4,4 | 3,3 | 2,9 | 1,4 | 1,4 | 0,9 | 1,0 | 1,1 | 0,4 | 0,5 | 0,1 | 0,0 | 0,0 | 10,2 | 4,7 | 2,5 |
| sub 3 | 0,0 | 0,0 | 0,0 | 0,0 | 0,1 | 0,1 | 0,7 | 1,0 | 2,9 | 1,9 | 1,3 | 0,9 | 1,0 | 0,8 | 0,9 | 0,3 | 0,3 | 0,1 | 0,2 , | 0,3 | 0,2 | 0,0 | 0,0 | 5,2 | 2,9 | 2,0 |
| sub 4 | 0,0 | 0,0 | 1,5 | 16,7 | 25,9 | 25,3 | 24,6 | 26,4 | 24,0 | 18,1 | 10,5 | 14,5 | 10,9 | 3,8 | 4,1 | 3,3 | 3,0 | 2,7 | 2,0 | 1,9 | 1,1 | 1,1 | 0,4 | 74,9 | 26,4 | 1,8 |
| sub 5 | 0,0 | 0,0 | 0,0 | 0,0 | 0,0 | 0,0 | 0,0 | 0,2 | 0,5 | 1,0 | 1,5 | 1,8 | 2,0 | 2,4 | 2,8 | 1,9 | 2,5 | 2,3 | 0,9 | 0,4 | 0,2 | 0,0 | 0,0 | 8,2 | 2,8 | 4,3 |
| sub 6 | 0,0 | 0,4 | 0,6 | 5,2 | 11,3 | 21,9 | 20,6 | 22,7 | 16,4 | 8,7 | 7,3 | 5,4 | 4,2 | 4,1 | 4,4 | 2,7 | 3,0 | 2,9 | 2,3 | 2,0 | 1,8 | 0,8 | 0,4 | 52,3 | 22,7 | 1,8 |
| Mean | 0,0 | 0,2 | 0,7 | 7,4 | 12,6 | 16,9 | 18,1 | 19,8 | 17,3 | 13,9 | 10,6 | 9,9 | 7,9 | 5,1 | 5,6 | 4,0 | 4,2 | 4,0 | 2,6 | 2,1 | 1,3 | 0,7 | 0,4 | 59,3 | 22,8 | 2,3 |
| SD | 0,0 | 0,2 | 0,6 | 6,7 | 10,5 | 11,9 | 11,0 | 11,8 | 9,5 | 6,2 | 3,6 | 4,9 | 3,6 | 1,3 | 1,4 | 1,2 | 1,1 | 1,1 | 0,9 | 0,8 | 0,7 | 0,5 | 0,2 | 28,3 | 10,5 | 1,0 |

| **Fenofibrate Plasma Levels [µg/ml]** | | | | | | | | | | | | | | | | | | | | | | | | | | |
|---|---|---|---|---|---|---|---|---|---|---|---|---|---|---|---|---|---|---|---|---|---|---|---|---|---|---|
| samples | T0 | T1 | T2 | T3 | T4 | T5 | T6 | T7 | T8 | T9 | T10 | T11 | T12 | T13 | T14 | T15 | T16 | T17 | T18 | T19 | T20 | T21 | T22 | | | |
| Hours | 0,0 | 0,3 | 0,5 | 0,8 | 1,0 | 1,3 | 1,5 | 1,8 | 2,0 | 2,5 | 3,0 | 3,5 | 3,8 | 4,0 | 4,3 | 4,5 | 4,8 | 5,0 | 6,0 | 7,3 | 8,0 | 10,0 | 12,0 | AUCt | Cmax | Tmax |
| sub 1 | 0,0 | 0,0 | 0,0 | 0,0 | 0,0 | 0,3 | 1,1 | 2,4 | 3,2 | 4,4 | 4,8 | 4,6 | 4,4 | 3,9 | 4,9 | 4,9 | 5,5 | 5,7 | 10,2 | 9,2 | 6,7 | 5,3 | 4,4 | 62,8 | 10,2 | 6,0 |
| sub 2 | 0,0 | 0,0 | 0,0 | 0,0 | 0,0 | 0,0 | 0,0 | 0,0 | 0,2 | 1,1 | 2,1 | 3,2 | 3,9 | 6,3 | 6,6 | 8,0 | 7,3 | 8,8 | 10,3 | 6,8 | 10,9 | 8,2 | 7,5 | 73,8 | 10,9 | 8,0 |
| sub 3 | 0,0 | 0,0 | 0,0 | 0,0 | 0,3 | 0,6 | 1,0 | 1,9 | 3,3 | 5,9 | 6,9 | 6,2 | 6,4 | 5,5 | 7,3 | 8,1 | 8,0 | 9,3 | 8,5 | 9,0 | 6,2 | 6,6 | 6,6 | 72,4 | 9,3 | 5,0 |
| ,sub 4, | 0,0 | 0,0 | 0,0 | 0,5 | 1,7 | 2,9 | 3,6 | 4,5 | 4,8 | 5,8 | 7,0 | 8,3 | 9,3 | 8,5 | 8,7 | 8,0 | 7,4 | 6,9 | 6,8 | 5,2 | 5,2 | 4,1 | 3,5 | 60,9 | 9,3 | 3,8 |
| sub 5 | 0,0 | 0,0 | 0,0 | 0,0 | 0,0 | 0,2 | 0,3 | 0,4 | 0,5 | 1,0 | 1,6 | 2,5 | 3,7 | 4,6 | 5,4 | 7,2 | 9,2 | 8,5 | 9,0 | 8,1 | 6,0 | 6.0 | 5,5 | 59,3 | 9,2 | 4,8 |
| sub 6 | 0,0 | 0,0 | 0,0 | 0,0 | 1,0 | 3,9 | 7,4 | 10,9 | 10,9 | 11,8 | 10,9 | 10,4 | 9,7 | 9,4 | 9,1 | 8,9 | 8,5 | 7,6 | 7,4 | 6,2 | 5,1 | 4,1 | 3,4 | 74,5 | 11,8 | 2,5 |
| average | 0,0 | 0,0 | 0,0 | 0,1 | 0,5 | 1,3 | 2,2 | 3,3 | 3,8 | 5,0 | 5,5 | 5,9 | 6,2 | 6,4 | 7,0 | 7,5 | 7,6 | 7,8 | 8,7 | 7,4 | 6,7 | 5,7 | 5,1 | 67,3 | 10,1 | 5,0 |
| SD | 0,0 | 0,0 | 0.0 | 0,2 | 0,7 | 1.7 | 2,8 | 4,0 | 3,9 | 4,0 | 3,5 | 3,1 | 2,7 | 2,2 | 1,7 | 1,4 | 1,2 | 1,3 | 1,4 | | 2,2 | 1,6 | 1,7 | 7,0 | 1,1 | 1,9 |

It clearly appears that the invention allows indeed, to avoid simultaneous plasma peaks of both active ingredients, so allowing to minimize the risk of side effects due to the combination of drugs. Indeed, when the mean Tₘₐₓ of fenofibric acid occurs (5 hours), the mean plasma concentration of pravastatin has already decreased to 4.0 ng/ml. Furthermore, the mean Tₘₐₓ of fenofibric acid and of pravastatin are sufficiently separated. Indeed, the absolute difference of |mean Tₘₐₓ fenofibric acid - mean Tₘₐₓ pravastatin | is equal to |5.0 - 2.3| or 2.7 hours.

### Example 5

| **Ingredient Name** | **Amount [g]** |
|---|---|
| Pravastatin Sodium Salt | 40 |
| Sodium bicarbonate | 112 |
| Lactose monohydrate | 19 |
| Cellulose microcrstalline | 12 |
| Povidone K30 | 2 |
| Water for granulation | 30 |
| Magnesium stearate | 2 |
| Sodium starch glycolate | 13 |

Lactose, sodium bicarbonate, lactose monohydrate, cellulose microcrystalline and povidone K30 were blended in a planetary mixer for about 5 to 10 minutes until an homogeneous blend was obtained. While under agitation, a suspension containing the pravastatin sodium salt into the water for granulation was added to granulate the powders. The granules obtained were dried at about 40°C for about 5 hours. After the dried granules were screened through a 1.0mm sieve, they were blend into a planetary mixer for about 2 minutes after the addition of the magnesium stearate and sodium starch glycolate. The final mix was compressed into tablets using a rotary compressing machine equipped with punches of the deep cup type with a diameter of 6.5mm. The mean weight of the tablets was 200 mg. Corresponding to tablets containing 40 mg of pravastatin sodium salt. The tablets had hardness comprised between 4 and 6 kilopascals (Kp).

When a core tablet was placed into 100ml of demineralized water a pH of about 8.3 was measured.

These core tablets were coated with the coating solution and the coating method parameters of Example 2.

### Example 6

| **Ingredient** | **Amount [g]** |
|---|---|
| Fenofibrate powder | 160 |
| Lactose | 300 |
| Povidone K30 | 15 |
| Sodium Lauryl Sulfate | 7 |
| Crospovidone | 15 |
| Magnesium Stearate | 3 |

Fenofibrate, lactose, povidone and sodium lauryl sulfate were blended in a planetary mixer and water was added to granulate. After oven drying for about 5 hours at 50°C, the granules were screened through a 1mm sieve. After addition of crospovidone and the magnesium stearate the granules that were blended for an additional 3 minutes in the planetary mixer.

### Example 7

500 mg of lubricated granules of Example 6 and a tablet of Example 5 were filled into 0 elongated hydroxypropylmethylcellulose capsules to produce a combination product containing 40mg of pravastatin and 160mg of fenofibrate.

### Example 8

| **Ingredient Name** | **Amount [g]** |
|---|---|
| Pravastatin sodium salts | 10 |
| Sodium bicarbonate | 40 |
| Microcrystalline cellulose | 100 |
| Povidone K30 | 20 |
| | 170 |

To the blend of all the ingredients in a planetary mixer was added water to granulate. The paste obtained was extruded and spheronized in order to obtain beads with a diameter of about 1 mm. The beads were tray dried in an oven at about 40°C for approximately 5 hours. The beads were thereafter screened between 0.7mm and 1.4mm sieves.

### Example 9

500 g of beads from Example 8 were coated with 200 g of coating solution (which are equal to 40 g of dry residue) of Example 2 using a fluid bed coater (Strea 1) equipped with a wurster column.

### Example 10

A combination formulation was produced by filling 0 elongated hard gelatin capsules with 500 mg of Fenofibrate lubricated granules of Example 6 and 170 mg of Pravastatin beads of Example 8.

The resulting combination formulation contained 10mg of pravastatin and 160mg of fenofibrate.

### Example 11

Tablets of Pravastatin sodium salt with the composition as described in Table1 were prepared following the method of Example 1 and thereafter were coated with the insulation coating of Example 2.

From each of the tablets was produced a combination products by filling 0 elongated hard gelatin capsules with 564mg of the hot blend of Example 3and a coated tablet as described in Table.

Each of these combination products were packaged in high density polyethylene bottles with a desiccant.

The packaged product underwent a stability study under accelerated conditions at 40°C/75% relative humidity. The Pravastatin, Pravastatin Lactose and other impurities were measured by HPLC before and after 1 and 3 months of storage.

As a comparison the marketed tablets in Belgium Pravasine® 40 mg (Brystol Myers Squibb) have been stored in the same packaging. The Pravasine^{®} 40 mg tablets correspond to the US patent 5,030,447 which claims to stabilize the pravastatin in a tablet form by using excipients able to produce a pH above 9 when the tablet is dissolved in water.

The results of stability are presented in the Table 3.

| **Table 1. Composition of Combination Product** | | | | | |
|---|---|---|---|---|---|
| | Ingredient Amount [mg / tablet and capsule] | | | | |
| Ingredient Name | 11A | 11B | 11C | 11D | 11E |
| A. Pravastatin Tablet | | | | | |
| pravastatin Na | 40 | 40 | 40 | 40 | 20 |
| Na bicarbonate | 112 | 110 | 80 | / | / |
| Ca hydrogenophosphate | / | / | / | 110 | / |
| ascorbyl palmitate | / | 2 | / | / | / |
| Lactose | 19 | 19 | 25 | 19 | 24 |
| Microcrystalline cellulose | 12 | 12 | 38 | 12 | 18.4 |
| Polyvinylpyrrolidone | 2 | 2 | 2 | 2 | |
| sodium starch glycolate | 13 | 13 | 13 | 13 | 14.4 |
| magnesium stearate | 2 | 2 | 2 | 2 | 3.2 |
| mannitol | / | / | / | / | / 112 |
| Insulation Coating | yes | yes | yes | yes | yes |
| pH of 1 tablet in 100mL | 8.3 | 8.3 | 8.1 | 7.3 | 6.0 |
| of water | | | | | |

| B. Fenofibrate | | | | | |
|---|---|---|---|---|---|
| Fenofibrate | 160 | | | | |
| Gelucire 44/14 | 240 | | | | |
| PEG 20,000 | 48 | | | | |
| Hydroxypropylcellulose | 95.0 | | | | |
| Sodium starch glycolate | 20.0 | | | | |
| Lauryl palmitate | 1.00 | | | | |
| 0 elongated hard | 1 capsule | | | | |
| gelatin capsules | | | | | |

**Table 3: Stability of different batches of pravastatin tablets when combined with fenofibrate.**

| | After preparation | After 1 month | After 3 month |
|---|---|---|---|
| **Example 11A** | | | |
| Pravastatin | 98.8 | 99.4 | 98.6 |
| Pravastatin lactone | ND | ND | ND |
| other impurities | ND | 0.07 | 0.12 |

| **Example 11B** | | | |
|---|---|---|---|
| Pravastatin | 98.9 | 100.7 | 100.8 |
| Pravastatin lactone | ND | ND | ND |
| other impurities | 0.05 | 0.05 | 0.10 |

| **Example 11C** | | | |
|---|---|---|---|
| Pravastatin | 99.5 | 98.8 | 97.7 |
| Pravastatin lactone | ND | ND | 0.20 |
| other impurities | 0.07 | 0.09 | 0.11 |

| **Example 11 D** | | | |
|---|---|---|---|
| Pravastatin | 102.4 | 100.2 | 99.2 |
| Pravastatin lactone | ND | 0.34 | 1.18 |
| other impurities | ND | 0.07 | 0.28 |

| **Example 11 E** | | | |
|---|---|---|---|
| Pravastatin | 98.0 | NP | 85.1 |
| Pravastatin lactone | ND | NP | 9.8 |
| other impurities | 0.12 | N"P | 2.1 |

| **PRAVASINE 40 mg** | | | |
|---|---|---|---|
| Pravastatin | 103.7 | 103.2 | 103.5 |
| Pravastatin lactone | ND | ND | 0.11 |
| other impurities | ND | 0.22 | 0.25 |

| | | | |
|---|---|---|---|
| ND : Not detected NP: Not performed | | | |

- the use of a alkaline agent conferring a pH less than 9 significantly improves the stability of pravastatin tablets since the batch containing no alkaline agent (Example 11E) has a lactone content of 9.8% after 3 months which is about 50 to 100 times more than the formulation containing such alkaline agent.
- the stability observed with the formulations of the present invention with tablets of pravastatin presenting a pH comprised between 7 to 9 presents stability properties which are at least as good, if not better, than the marketed product PRAVASINE^{®}, which presents the inconvenient of having a pH, when dissolved in water, of about 10. As said before, these high pH values are not very well compatible with the stomach environment.
- for pravastatin tablets containing sodium bicarbonate, it also appears that the amount of sodium bicarbonate per tablet is important since the tablets containing 112 mg of sodium bicarbonate (Example 11A) present slightly better stability results than the formulation containing 80 mg of sodium bicarbonate / tablet (Example 11 C).

The results of the present stability shows clearly that tablets prepared following the teachings of the present invention possesses very good stability characteristics while avoiding the need to use very alkaline substances as disclosed in US Patent 5,030,447 and 5,180,589.

### Example 12

The example 12 is similar to example 1 where 1 g of folic acid has been added to the pravastatin component of the present combination formulation.

| **Ingredient Name** | **Amount [g]** |
|---|---|
| Pravastatin Sodium Salt | 20 |
| Sodium bicarbonate | 110 |
| folic acid | 1 |
| Ascorbyl palmitate | 2 |
| Lactose monohydrate | 19 |
| Cellulose microcrystalline | 12 |
| Povidone K30 | 2 |
| Water for granulation | 25 |
| Magnesium stearate | 2 |
| Sodium starch glycolate | 13 |

### Example 13

example 13 is similar to example 3 but where 3 mg of folic acid has been added to the fenofibrate component of the present combination formulation.

| **Ingredient Name** | **Amount [g]** |
|---|---|
| Fenofibrate powder | 160 |
| Lauroyl macrogolglyceride | 240 |
| (gelucire 44/14) | |
| Polyethylene glycol 20,000 | 48 |
| Hydroxypropylcellulose | 95.0 |
| folic acid | 3.0 |
| Sodium starch glycolate | 20.0 |
| Ascorbyl palmitate | 1.0 |

## Claims

1. A pharmaceutical composition comprising effective amounts of:
(a) Pravastatin and/or a pharmaceutical acceptable salt thereof;
(b) Fenofibrate; and
(c) sodium bicarbonate in an amount to confer a pH of less than 9 when dissolved and/or dispersed in water.

2. A composition according to claim 1 further containing a substance from the group of vitamins, minerals, nutrients and mixtures thereof.

3. A composition according to claim 2 where the vitamin derivative is folic acid, vitamin 86 or vitamin B12 or a mix of two or more of those vitamins

4. A composition according to claim 1 further containing between 0.05 and 100 mg of folic acid

5. A composition according to claim 1 containing an amount of Fenofibrate comprised between 5 and 300mg, advantageously between 25 mg and 200 mg.

6. A composition according to claim 1 containing an amount of Pravastatin, and/or a pharmaceutical acceptable salt thereof comprised between 5 mg and 120 mg.

7. A composition according to claim 1 containing an amount of Pravastatin and/or a pharmaceutical acceptable salt thereof comprised between 10mg and 80mg.

8. An oral composition according to any of the preceding claims **characterized in that** it is presented under the form of an hard gelatin, or hypromellose or any other pharmaceutically acceptable capsule.

9. A composition according to any of the preceding claims **characterised** that the combination product is presented under the form of a tablet.

10. A compositon according to any of the preceding claims **characterised** that the Fenofibrate is present in the form of a semi-solid paste.

11. A composition according to claim 10 **characterised in that** the Fenofibrate is dissolved and/or dispersed into a polyglyceride.

12. A composition according to any claims 1 to 9 **characterised in that** the Fenofibrate is present in a micronized form.

13. A composition according to claim 12 **characterised in that** the micronized Fenofibrate is coated onto an hydrosoluble carrier.

14. A composition according to claim 12 **characterised in that** the Fenofibrate is co-micronized with a surfactant.

15. A composition according to claim 12 **characterised in that** the Fenofibrate is blended and/or granulated with a surfactant

16. A composition according to any of the preceding claims **characterised in that** it further comprises an antioxidant agent.

17. A composition according to claim 16 wherein the antioxidant is selected from the ascorbic acid, ascorbic acid derivatives, vitamins E, vitamin E derivatives, propylgallate, butylhydroxyanisole, butylhydroxytoluene, sulfite.

18. A composition according to claim 16 wherein the antioxidant is ascorbyl palmitate.

19. A composition according to any of the preceding claims **characterized in that** it may be administered in the morning or in the evening.

20. The composition according to any of the preceding claims which is useful for the treatment of hypercholesterolemia and/or hyperlipidemia,

21. Use of Pravastatin and/or an acceptable salt thereof, fenofibrate and sodium bicarbonate for the preparation of a composition for treatment of hypercholesterolemia and/or hyperlipidemia with low risk of side-effects.

## Patentansprüche

1. Pharmazeutische Zusammensetzung, die wirksame Mengen umfasst von:
(a) Pravastatin und/oder einem pharmazeutisch verträglichen Salz davon;
(b) Fenofibrat; und
(c) Natriumhydrogencarbonat in einer Menge, um einen pH-Wert von unter pH 9 zu verleihen, wenn es in Wasser gelöst und/oder fein verteilt ist.

2. Zusammensetzung nach Anspruch 1, die ferner eine Substanz aus der Gruppe der Vitamine, Mineralien, Nährstoffe und Mischungen daraus enthält.

3. Zusammensetzung nach Anspruch 2, wobei das Vitamin-Derivat Folsäure, Vitamin B6 oder Vitamin B12 oder eine Mischung aus zwei oder mehr von diesen Vitaminen ist.

4. Zusammensetzung nach Anspruch 1, die ferner zwischen 0,05 und 400 mg Folsäure enthält.

5. Zusammensetzung nach Anspruch 1, die eine Fenofibrat-Menge umfasst, die zwischen 5 und 300 mg, vorzugsweise zwischen 25 mg und 200 mg liegt.

6. Zusammensetzung nach Anspruch 1, die eine Menge an Pravastatin und/oder einem pharmazeutisch verträglichen Salz davon umfasst, die zwischen 5 mg und 120 mg liegt.

7. Zusammensetzung nach Anspruch 1, die eine Menge an Pravastatin und/oder einem pharmazeutisch verträglichen Salz davon umfasst, die zwischen 10 mg und 80 mg liegt.

8. Orale Zusammensetzung nach einem der vorangehenden Ansprüche, **dadurch gekennzeichnet, dass** sie in der Form von einer Hartgelatinekapsel oder einer aus Hypromellose hergestellten Kapsel oder irgendeiner anderen pharmazeutisch verträglichen Kapsel dargeboten wird.

9. Zusammensetzung nach einem der vorangehenden Ansprüche, **dadurch gekennzeichnet, dass** das Kombinationsprodukt in Form einer Tablette dargeboten wird.

10. Zusammensetzung nach einem der vorangehenden Ansprüche, **dadurch gekennzeichnet, dass** Fenofibrat in Form einer halbfesten Paste vorliegt.

11. Zusammensetzung nach Anspruch 10, **dadurch gekennzeichnet, dass** das Fenofibrat in einem Polyglycerid gelöst und/oder fein verteilt ist.

12. Zusammensetzung nach den Ansprüchen 1 bis 9, **dadurch gekennzeichnet, dass** das Fenofibrat in einer mikronisierten Form vorliegt.

13. Zusammensetzung nach Anspruch 12, **dadurch gekennzeichnet, dass** das mikronisierte Fenofibrat auf ein wasserlösliches Trägermaterial aufgetragen ist.

14. Zusammensetzung nach Anspruch 12, **dadurch gekennzeichnet, dass** das Fenofibrat mit einem Netzmittel co-mikronisiert ist.

15. Zusammensetzung nach Anspruch 12, **dadurch gekennzeichnet, dass** das Fenofibrat mit einem Netzmittel vermischt und/oder granuliert ist.

16. Zusammensetzung nach einem der vorangehenden Ansprüche, **dadurch gekennzeichnet, dass** sie ferner ein Antioxidationsmittel umfasst.

17. Zusammensetzung nach Anspruch 16, wobei das Antioxidationsmittel ausgewählt ist aus Ascorbinsäure, Ascorbinsäurederivaten, Vitamin E, Vitamin-E-Derivaten, Propylgallat, Butylhydroxyanisol, Butylhydroxytolul, Sulfit.

18. Zusammensetzung nach Anspruch 16, wobei das Antioxidationsmittel Ascorbylpalmitat ist.

19. Zusammensetzung nach einem der vorangehenden Ansprüche, **dadurch gekennzeichnet, dass** sie am Morgen oder am Abend verabreicht werden kann.

20. Zusammensetzung nach einem der vorangehenden Ansprüche, die für die Behandlung von Hypercholesterinämie und/oder Hyperlipidämie verwendbar ist.

21. Verwendung von Pravastatin und/oder einem verträglichen Salz davon, von Fenofibrat und von Natriumhydrogencarbonat für die Zubereitung von einer Zusammensetzung für die Behandlung von Hypercholesterinämie und/oder Hyperlipidämie mit einem geringen Risiko für Nebenwirkungen.

## Revendications

1. Composition pharmaceutique comprenant des quantités efficace de :
(a) pravastine et/ou de l'un de ses sels pharmaceutiquement acceptable ;
(b) fénofibrate ; et
(c) bicarbonate de sodium en une quantité permettant de conférer un pH inférieur à 9 quand elle est dissoute et/ou dispersée dans l'eau.

2. Composition selon la revendication 1, contenant en outre une substance provenant du groupe des vitamines, des minéraux, des nutriments et de leurs mélanges.

3. Composition selon la revendication 2, où le dérivé de vitamine est l'acide folique, la vitamine B6 ou la vitamine B12 ou un mélange de deux ou de plus de deux de ces vitamines.

4. Composition selon la revendication 1, contenant en outre entre 0,05 mg et 100 mg d'acide folique.

5. Composition selon la revendication 1, contenant une quantité de fénofibrate comprise entre 5 et 300 mg, de manière avantageuse entre 25 mg et 200 mg.

6. Composition selon la revendication 1, contenant une quantité de pravastine et/ou de l'un de ses sels pharmaceutiquement acceptables comprise entre 5 mg et 120 mg.

7. Composition selon la revendication 1, contenant une quantité de pravastine et/ou de l'un de ses sels pharmaceutiquement acceptables comprise entre 10 mg et 80 mg.

8. Composition orale selon l'une quelconque des revendications précédentes, **caractérisée en ce qu'**elle se présente sous la forme d'une capsule de gélatine dure ou d'hypromellose ou de n'importe quelle autre capsule pharmaceutiquement acceptable.

9. Composition selon l'une quelconque des revendications précédentes, **caractérisée en ce que** le produit en combinaison se présente sous la forme d'un comprimé.

10. Composition selon l'une quelconque des revendications précédentes, **caractérisée en ce que** le fénofibrate se présente sous la forme d'une pâte semi-solide.

11. Composition selon la revendication 10, **caractérisée en ce que** le fénofibrate est dissous et/ou dispersé dans un polyglycéride.

12. Composition selon l'une quelconque des revendications 1 à 9, **caractérisée en ce que** le fénofibrate est présent sous une forme micronisée.

13. Composition selon la revendication 12, **caractérisée en ce que** le fénofibrate micronisé est enrobé sur un support hydrosoluble.

14. Composition selon la revendication 12, **caractérisée en ce que** le fénofibrate est co-micronisé avec un tensioactif.

15. Composition selon la revendication 12, **caractérisée en ce que** le fénofibrate est mélangé et/ou mis sous forme de granulés avec un tensioactif.

16. Composition selon l'une quelconque des revendications précédentes, **caractérisée en ce qu'**elle comprend en outre un agent antioxydant.

17. Composition selon la revendication 16, dans laquelle l'antioxydant est choisi parmi l'acide ascorbique, les dérivés de l'acide ascorbique, la vitamine E, les dérivés de la vitamine E, le gallate de propyle, le butylhydroxyanisole, le butylhydroxytoluène, un sulfite.

18. Composition selon la revendication 16, dans laquelle l'antioxydant est le palmitate d'ascorbyle.

19. Composition selon l'une quelconque des revendications précédentes, **caractérisée en ce qu'**elle peut être administrée le matin ou le soir.

20. Composition selon l'une quelconque des revendications précédentes, qui est utile pour le traitement de l'hypercholestérolémie et/ou de l'hyperlipidémie.

21. Utilisation de pravastine et/ou de l'un de ses sels acceptables, de fénofibrate et de bicarbonate de sodium pour la préparation d'une composition destinée au traitement de l'hypercholestérolémie et/ou de l'hyperlipidémie avec peu de risque d'effets secondaires.
